# EUROPEAN PATENT SPECIFICATION

(11) **EP 0 356 264 B1**
(45) Date of publication and mention of the grant of the patent: **12.10.1994**
(21) Application number: 89308792.4
(22) Date of filing: 22.08.1989
(51) Int. Cl.: A01N 47/44, C11D 3/00, C11D 3/48

(54) **Cleansing composition for topical disinfection**
Reiningungsmittel für die örtliche Desinfektion
Composition nettoyante pour la désinfection locale

(30) Priority: 22.08.1988 US 234706
(43) Date of publication of application: 28.02.1990
(73) Proprietor: Becton, Dickinson and Company, Franklin Lakes, New Jersey 07417-1880 (US)
(72) Inventor: Khan, Mohammed A., Sandy Utah (US); Moellmer, John F., Salt Lake City Utah (US)
(74) Representative: Ruffles, Graham Keith

(56) References cited:
- EP-A- 0 231 080
- WO-A-86/02090
- WO-A-87/00005
- GB-A- 2 200 845
- US-A- 4 420 484

## Description

This invention relates to antimicrobial activity, and, more specifically, relates to antimicrobial cleansing compositions including chlorhexidine and a nonionic surfactant.

The antimicrobial effects of bisbiguanides have long been known. Chlorhexidine is the best known member of the class, and this product has been marketed for many years in various formulations such as antibacterial hand washes and surgical scrub compositions. These formulations generally include both a surface active agent and a low percentage of an alcohol, usually isopropanol.

Burdon et al. reported in 1967 that stock solutions of chlorhexidine frequently were contaminated with species of Pseudomonas, but that the combination of chlorhexidine and 4% V/V isopropanol greatly reduced this problem. Nevertheless, the authors speculated that continued use of isopropanol may ultimately result in selection of strains resistant to the chlorhexidine-isopropanol combination.

Billany et al., in U.S. Patent No. 3,960,745, discloses a chlorhexidine cleansing composition formulated with a polyoxyethylene-polyoxypropylene nonionic sufactant. The Billany et al. formulation is marketed under the trade name Hibiclens^{R} by Stuart Pharmaceuticals, Wilmington, Delaware, a division of ICI Americas Inc. Billany et al. teaches that anionic, cationic and amphoteric surfactants all form complexes with chlorhexidine, and that of 17 nonionic surfactants studied, only four, all polyoxyethylene-polyoxypropylene surfactants, could be formulated with chlorhexidine with retention of 70% of the antimicrobial activity of a 2% solution of chlorhexidine gluconate. The patent further teaches that not even all members of this class are equally suitable for chlorhexidine formulations, and that complexation of the chlorhexidine with the surfactant results in a substantial reduction of the antibacterial activity of the chlorhexidine.

U.S. Patent No. 4,420,484 to Gorman et al. discloses a skin cleansing composition consisting of a bisbiguanide antimicrobial agent and a combination of surfactants formulated with water, alcohol and various other ingredients. The Gorman et al. patent states that all ingredients in the patented composition are particularly described in the prior art.

Owens, in U.S Patent No. 4,456,543 shows an antibacterial cleansing product containing a bisbiguanide and one or more nonionic polyoxyalkylene surfactants containing oxyethylene, oxypropylene and oxybutylene blocks. Owens, like Billany et al., states that complexation of chlorhexidine and the surfactant results in a substantial reduction of antibacterial activity.

WO 86/2090 describes an antiseptic cleansing composition comprising a salt of chlorhexidine and a nonionic surfactant. The composition does not, however, include all of the components hereinafter described. WO 87/00005 also describes a chlorhexidine containing composition, with low concentrations of chlorhexidine and TRIS buffer providing a stable composition.

Chlorhexidine-containing compositions are marketed by Stuart Pharmaceuticals, Wilmington, Delaware, under the trade name Hibiclens^{R}; by Xtrium Laboratories, Inc., Chicago, Illinois, under the trade name Exidine^{R}, by Medical Systems Research, Inc., Salt Lake City, Utah, under the trade name Steri-Stat and by Huntington Laboratories, Inc., Huntington, Indiana, under the trade name Cida-Stat.

Chlorhexidine cleansing compositions are used principally as hand washes and surgical scrubs. As such, it is desirable to effect the most complete kill possible of the bacterial flora which routinely proliferate on the skin. The principal organism existing on the skin is Staphylococcus aureus, an organism well-known to be resistant to antibacterial agents. Accordingly, there is a need for a chlorhexidine composition particularly effective against this organism. This invention addresses this and other needs.

### SUMMARY OF THE INVENTION

An antimicrobial cleansing composition includes a salt of chlorhexidine and a nonylphenoxypoly(ethyleneoxy)ethanol surfactant in an aqueous vehicle. The preferred salt is the gluconate and is included in the composition in a concentration of about 4% by weight. (In the present disclosure, all percentages are by weight unless otherwise stated.) Other surfactants and thickening agents such as polyethyleneglycol (hereinafter PEG) esters of fatty acids, PEG ethers of lanolin and fatty acid amides are included in the composition. Other ingredients such as dyes and perfumes may be added to give the composition any desired color and scent. The most preferred vehicle is water, and the pH may be adjusted to any desired level by adding acid or base as required.

All surfactants in chlorhexidine compositions are known to form complexes to a greater or lesser extent with the chlorhexidine. Chlorhexidine has long been believed to be deactivated by complexation wherein antibacterial activity resides only in that portion of the chlorhexidine which is not complexed. The composition of the present invention is formulated with a surfactant heretofore not disclosed in chlorhexidine formulations. The surfactant and chlorhexidine of the present composition are highly complexed, yet, in contrast to prior art reports, the formulation is highly effective, providing substantially total kill of S. aureus and other bacteria.

### DETAILED DESCRIPTION

While this invention is satisfied by embodiments in many different forms, there will herein be described in detail preferred embodiments of the invention, with the understanding that the present disclosure is to be considered as exemplary of the principles of the invention and is not intended to limit the invention to the embodiments described. The scope of the invention will be measured by the appended claims and their equivalents.

The chlorhexidine in the present cleansing composition is 79% complexed with a particular surfactant, yet provides rapid and substantially complete kill of most bacteria, including S. aureus. In addition, the present composition provides all the other attributes of known chlorhexidine formulations, such as safety, mildness, emolliency, and sudsing. The advantages of the present composition are consequent to incorporation into the composition of a nonylphenoxypoly(ethyleneoxy)ethanol surfactant.

The concentration of chlorhexidine in the composition of the present invention is from 2 to 6%, most preferably 3.5 to 4.5%. Chlorhexidine base may be used, however a salt of chlorhexidine which is soluble in the formulation is preferred. Preferred salts are the hydrochloride, acetate, and most preferably, the gluconate. Chlorhexidine gluconate is commercially available from ICI Americas, Inc., Wilmington, Delaware.

A nonionic surfactant of the nonylphenoxypoly(ethyleneoxy)ethanol type is included in the composition of the invention. This class of surfactants is commercially available from GAF Corporation, Wayne, New Jersey, under the trade name Igepal^{R}, and has the following formula:

C₉H₁₉- Ø - O - (CH₂CH₂ O) ₙ₋₁ CH₂CH₂OH

where n is the number of molecules of ethylene oxide per molecule of nonylphenol. Preferred Igepal^{R} surfactants have about 60 to 80, preferably about 66 to 75% ethylene oxide. The most preferred Igepal^{R} surfactant of the invention is Igepal^{R} CO-720 having about 71% ethylene oxide. The surfactant is present in the composition in a concentration of 2 to 10%, preferably 4 to 6%, most preferably about 5% of the total weight of the composition.

In addition to the Igepal^{R} surfactant, the composition of the invention includes additional nonionic surfactants. This class of surfactants is also commercially available, and may be obtained from Amerchol Corporation, Edison, New Jersey, under the trade name Solulan^{R}. Preferred Solulan^{R} surfactants have hydroxyl values of about 35 to 75. The most preferred Solulan^{R} surfactant is Solulan^{R} 75 having a hydroxyl value of 40-50. This product confers emulsifying and plasticizing properties to the composition and, in addition, being soluble in water, aids in solubilizing or dispersing other ingredients of the compositions. The quantity of the Solulan^{R} surfactant in the composition may advantageously be from 3 to 7%, preferably 4.5 to 5.5%, most preferably about 5%.

Other ingredients which are conventional or desirable in various cosmetic formulations are added to the composition of the invention. For instance, one or more thickening agents are advantageous. Particularly useful thickening agents are fatty acid esters of PEG having a molecular weight of about 200 to 6000. For example, PEG esters of lauric acid, oleic acid, and, most preferably stearic acid, such as PEG-6000 distearate may be used. This product is commercially available from Stepan Co., Northfield, Illinois, as Kessco^{R} PEG-6000. Fatty acid amide thickening agents may be used, such as ammonia, ethanolamine and diethanolamine amides of oleic acid, coco acid, or preferably, lauric acid. A particularly preferred thickening agent is lauric acid diethanolamide, commercially available from Witco Chemical Corporation Houston, Texas, under the trade name Witcamide^{R} 5195. Both of these products are used within a range of 2 to 5%, preferably 3 to 4%, most preferably about 3.5% and provide conditioning, emulsifying and foam stabilizing properties to the composition in addition to being thickeners.

If desired, the composition of the invention may include a perfume to provide a pleasing scent or a dye to provide a characteristic color. The preferred composition is colored red by inclusion of sufficient Red #40 to achieve the desired color. Most preferably, a concentration of about 0.01% of Red #40 is added to the composition.

It is preferred that the pH of the composition be adjusted to about 7.0 by addition of a suitable acidifying or alkalinizing agent, such as 6 N hydrochloric acid or 50% sodium hydroxide.

The present invention is more particularly described by means of, the following examples.

### EXAMPLE I

### Preferred Composition of the Invention

| | |
|---|---|
| Chlorhexidine gluconate | 4.1% |
| Igepal^{R} CO-720 | 5.0% |
| Solulan^{R} 75 | 5.0% |
| Witcamide 5195 | 3.5% |
| PEG-6000 distearate | 3.5% |
| Red #40 | 0.01% |
| Water | 78.89% |

### EXAMPLE II

### Method of Manufacture of the Composition of Example I

In a suitably sized vessel equipped for mixing was placed 61.18 g of purified water and 21.81 g of an 18.8% water solution of chlorhexidine gluconate B.P. After mixing well, 5.0 g of Igepal^{R} CO-720 was added slowly and mixed well. Solulan^{R} 75, 5.0 g, was heated to 55°C until melted and then added with thorough mixing. Witcamide^{R} 5195, 5.0 g, was melted by heating to 40°C and added with thorough mixing. PEG-6000 distearate, 3.5 g, was added and the mixture was vigorously mixed until complete homogeneity had been achieved and no flakes remained. Red #40 dye (10 mg) was added and the mixture was stirred until a clear, red, syrupy liquid was obtained. The mixture was adjusted if necessary to pH 7.0 by the addition of either 6 N HCl or 50% NaOH.

### EXAMPLE III

### Determination of Percentage of Complexation Between Chlorhexidine and Surfactant

This determination was made in accordance with the procedure of Owens, supra and gave the data summarized in Table I.

**TABLE I**

| Sample No. | 20 Hour Equilibration Time | | 72 Hour Equilibration Time | |
|---|---|---|---|---|
| | % Complexed | % Uncomplexed | % Complexed | % Uncomplexed |
| 1 | 26 | 74 | 13 | 87 |
| 2 | 79 | 21 | 72 | 28 |
| 3 | 0 | 100 | | |
| 4 | 73 | 27 | | |
| 5 | 77 | 23 | | |
| 6 | 82 | 18 | | |
| 7 | 79 | 21 | | |
| 8 | 86 | 14 | 83 | 17 |
| Key for sample number: 1 Hibiclens^{R} 2 Composition of Example I 3 4% chlorhexidine gluconate in water 4 Composition of Example I, but Igepal^{R} Co-720 replaced with Igepal^{R} CA-897 5 Composition of Example I, but Igepal^{R} Co-720 replaced with Igepal^{R} CO-710 6 Composition of Example I, but Igepal^{R} Co-720 replaced with Igepal^{R} CO-660 7 Composition of Example I, but Igepal^{R} Co-720 replaced with Igepal^{R} CA-630 8 Composition of Example I, but Solulan^{R} 75 replaced with Solulan^{R} 5 | | | | |

### EXAMPLE IV

### Efficacy Test

The composition of Example I was irradiated (3.1 Mrad) to ensure sterility and an efficacy comparison against Hibiclens^{R} was carried out by the following procedure:

Full strength composition (C), irradiated composition (C-I) and Hibiclens^{R} (H) were serially diluted 1:10; 1:100 and 1:1000. Each dilution was challenged with 0.1 ml of inoculum containing the number of colony forming units (CFU) of the 4 organisms given in Table II below. After exposure times of 1,2 and 5 minutes, 1.0 ml of each inoculated dilution was transferred to a tube containing 9 ml of Difco Dey Engley neutralizing broth. Samples (1.0 ml) of each dilution in neutralizing broth were further diluted into 9 ml of Difco Dey Engley neutralizing broth base. All tubes were then incubated at 30 to 35°C for 48 hours. Nutrient agar pour plates were prepared from each tube and examined for the presence of colonies after a minimum of 48 hours. The results of this experiment are given in Table II below.

**TABLE II**

| KILL TIMES (Minutes) | | | | | |
|---|---|---|---|---|---|
| ORGANISM | | DILUTIONS | H | C | C-I |
| 1. | Staphylococcus aureus | Full | Pos. | 1 | 1 |
| | 4.7X10⁶ CFU | 1:10 | Pos. | 1 | 1 |
| | | 1:100 | 2 | 1 | 1 |
| | | 1:1000 | Pos. | 5 | Pos. |
| 2. | Pseudomonas aeruginosa | Full | 1 | 1 | 1 |
| | 8.5X10⁶ CFU | 1:10 | 1 | 1 | 1 |
| | | 1:100 | 1 | 5 | 2 |
| | | 1:1000 | 5 | Pos. | 5 |
| 3. | Candida albicans | Full | 1 | 1 | 1 |
| | 3.10X10⁵ CFU | 1:10 | 1 | 1 | 1 |
| | | 1:100 | 1 | 1 | 1 |
| | | 1:1000 | 2 | 2 | 2 |
| 4. | Escherichia coli | Full | 1 | 1 | 1 |
| | 6.2X10⁶ CFU | 1:10 | 1 | 1 | 1 |
| | | 1:100 | 1 | 2 | 5 |
| | | 1:1000 | 5 | 2 | 5 |
| Pos. - colonies observed, total kill not achieved. | | | | | |

This test demonstrates that the composition of the invention was significantly more effective than Hibiclens^{R} versus S. aureus in spite of the fact that it is 79% complexed in contrast to Hibiclens which is only 26% complexed. The results versus the other organisms were identical through the 1:10 dilutions and similar at other dilutions. It is seen from Table II that irradiation of the composition did not significantly affect the antimicrobial efficacy of the composition, and that the irradiated composition is an effective antimicrobial cleansing composition.

Thus, the invention provides a cleansing composition which includes a chlorhexidine salt highly complexed with a nonionic nonylphenoxypoly(ethyleneoxy)ethanol surfactant. The composition of the invention including this particular surfactant has activity against S. aureus significantly greater than a prior art composition in which the degree of complexation is much lower. This result is completely unexpected in light of the heretofore generally accepted view that activity and complexation are inversely related. Since S. aureus is a commonly found organism on skin and is often difficult to kill completely, the composition of the invention represents a marked and unexpected improvement over prior art cleansing compositions.

## Claims

1. An antimicrobial cleansing composition comprising:
(a) 2 to 6% of a chlorhexidine salt;
(b) 4 to 6% of a nonylphenoxypoly(ethyleneoxy) ethanol surfactant;
(c) at least one additional ingredient which is 2 to 5% of a polyethyleneglycol diester of a first fatty acid and/or 2 to 5% of an amide of a second fatty acid;
(d) 3 to 7% of a polyethyleneglycol ether of a lanolin surfactant; and
(e) water.

2. A composition as claimed in claim 1 in which the salt is a hydrochloride, acetate or gluconate.

3. A composition as claimed in claim 1 in which the second fatty acid is stearic acid.

4. A composition as claimed in claim 1 in which the second fatty acid is lauric acid.

5. A composition as claimed in claim 4 in which the amide is an amide of ammonia, ethanolamine or diethanolamine.

6. A composition as claimed in any one of the preceding claims further comprising a dye.

7. A composition as claimed in any one of the preceding claims further comprising a pH adjusting compound which is an acid or a base.

8. An antimicrobial composition comprising:
(a) 4% of chlorhexidine gluconate;
(b) 5% of a nonylphenoxypoly(ethyleneoxy) ethanol;
(c) 5% of a polyethleneglycol ether of lanolin alcohol;
(d) 3.5% of a polyethyleneglycol distearate;
(e) 3.5% of lauric acid diethanolamide; and
(f) water.

9. A composition as claimed in claim 8 further comprising a dye.

10. A composition as claimed in claim 8 or claim 9 further comprising sufficient of a pH adjusting compound, which is an acid or a base, to adjust the pH of the composition to about 7.

## Patentansprüche

1. Antimikrobielles Reinigungsmittel, das folgende Komponenten umfaßt:
(a) 2 bis 6 % eines Chlorhexidinsalzes,
(b) 4 bis 6 % eines Nonylphenoxypoly(ethylenoxy)ethanol-Tensids,
(c) wenigstens eine zusätzliche Komponente, und zwar 2 bis 5% eines Polyethylenglycoldiesters einer ersten Fettsäure und/oder 2 bis 5% eines Amids einer zweiten Fettsäure,
(d) 3 bis 7% eines Polyethylenglycolethers eines Lanolin-Tensids und
(e) Wasser.

2. Mittel nach Anspruch 1, bei dem das Salz ein Hydrochlorid, Acetat oder Gluconat ist.

3. Mittel nach Anspruch 1, bei dem die zweite Fettsäure Stearinsäure ist.

4. Mittel nach Anspruch 1, bei dem die zweite Fettsäure Laurinsäure ist.

5. Mittel nach Anspruch 4, bei dem das Amid ein Ammoniak-, Ethanolamin- oder Diethanolaminamid ist.

6. Mittel nach einem der vorangegangenen Ansprüche, das außerdem noch einen Farbstoff enthält.

7. Mittel nach einem der vorangegangenen Ansprüche, das außerdem noch eine Verbindung zur Einstellung des pH's enthält, die eine Säure oder Base darstellt.

8. Antimikrobielles Mittel, das folgende Komponenten umfaßt:
(a) 4% Chlorhexidingluconat,
(b) 5% eines Nonylphenoxypoly(ethylenoxy)ethanols,
(c) 5% eines Polyetnylenglycolethers von Lanolinalkohol,
(d) 3,5% eines Polyethylenglycoldistearats,
(e) 3,5% eines Laurinsäurediethanolamids und
(f) Wasser.

9. Mittel nach Anspruch 8, das außerdem noch einen Farbstoff enthält.

10. Mittel nach Anspruch 8 oder 9, das außerdem noch eine ausreichende Menge einer Verbindung zur Einstellung des pH's des Mittels auf ca. 7 enthält, wobei diese eine Säure oder Base darstellt.

## Revendications

1. Composition nettoyante antimicrobienne comprenant:
(a) de 2 à 6% d'un sel de chlorhexidine;
(b) de 4 à 6% d'un nonylphénoxypoly(éthylènoxy)éthanol tensioactif;
(c) au moins un ingrédient additionnel qui est constitué de 2 à 5% d'un diester de polyéthylèneglycol d'un premier acide gras et/ou de 2 à 5% d'une amide d'un second acide gras;
(d) de 3 à 7% d'un éther polyéthylèneglycolique d'un agent tensioactif lanolinique; et
(e) de l'eau.

2. Composition telle que revendiquée dans la revendication 1, dans laquelle le sel est un chlorhydrate, un acétate ou un gluconate.

3. Composition telle que revendiquée dans la revendication 1, dans laquelle le second acide gras est l'acide stéarique.

4. Composition telle que revendiquée dans la revendication 1, dans laquelle le second acide gras est l'acide laurique.

5. Composition telle que revendiquée dans la revendication 4, dans laquelle l'amide est une amide de l'ammoniac, de l'éthanolamine ou de la diéthanolamine.

6. Composition telle que revendiquée dans l'une quelconque des revendications précédentes comprenant en outre une matière colorante.

7. Composition telle que revendiquée dans l'une quelconque des revendications précédentes comprenant en outre un agent d'ajustement du pH qui est un acide ou une base.

8. Composition antimicrobienne comprenant:
(a) 4% de gluconate de chlorhexidine;
(b) 5% d'un nonylphénoxypoly (éthylèneoxy)éthanol;
(c) 5% d'un éther polyéthylèneglycolique d'alcool de lanoline;
(d) 3.5% d'un distéarate de polyéthylèneglycol;
(e) 3.5% de diéthanolamide de l'acide laurique; et
(f) de l'eau.

9. Composition telle que revendiquée dans la revendication 8 comprenent en outre une matière colorante.

10. Composition telle que revendiquée dans la revendication 8 ou la revendication 9, comprenant en outre suffisamment d'un composé d'ajustement du pH qui est un acide ou une base pour ajuster à 7 environ le pH de la composition.
